# EUROPEAN PATENT APPLICATION

(11) **EP 2 105 150 A1**
(43) Date of publication of application: **30.09.2009**
(21) Application number: 08700770.4
(22) Date of filing: 14.01.2008
(51) Int. Cl.: A61L 31/18, A61L 31/16

(54) **DEVELOPABLE BIODEGRADABLE MICROSPHERIC BLOOD VESSEL EMBOLISM MATERIALS**

(30) Priority: 12.01.2007 CN 200710026270; 12.01.2007 CN 200710026271; 12.01.2007 CN 200710026273; 12.01.2007 CN 200710026274
(71) Applicant: Li, Yanfang, Guangdong 510060 (CN); Zhou, Taili, Guangdong 510060 (CN)
(72) Inventor: Li, Yanfang, Guangdong 510060 (CN); Zhou, Taili, Guangdong 510060 (CN)
(74) Representative: Messulam, Alec Moses
(86) International application number: PCT/CN2008/070100
(87) International publication number: WO 2008/086756

(57) **Abstract**

A radiopaque biodegradable vascular embolic microsphere comprises of a biodegradable material and a radiopaque material which can be both delivered to the vascular system, in which, the biodegradable material incorporates the radiopaque material to form granule structure. The embolic microsphere, readily visible under X-ray, can be delivered or drift with blood flow to a peripheral vessel or site, and can be manufactured using a simple process. A radiopaque biodegradable vascular embolic microsphere incorporating therapeutic agent(s) comprises of a biodegradable material, a radiopaque material, and therapeutic agent(s), which can be all delivered to the vascular system, in which, the biodegradable material incorporates the radiopaque material and the therapeutic agent to form granule structure. This embolic microsphere can be delivered to the targeted site under X-ray guidance, and then embolize the site, and release the therapeutic agent directly at the targeted site while degrading *in vivo*.

## Description

According to further features in preferred embodiments of the invention, the biodegradable material is made from polyesters, including polylactde, poly (lactide-co-glycolide), poly (lactide-ether) copolymer, and the blend of one or more of their derivatives.

According to further features in preferred embodiments of the invention, the biodegradable material is made from polycaprolactone, including polycaprolactone, poly (caprolactone-ether) block copolymer, polycaprolactone copolymer, poly (caprolactone-ether-lactide) copolymer, poly (glycolide-L-lactide) copolymer, poly (glycolide-L-lactide-caprolactone) copolymer, poly (caprolactone-caprolactone-glycolide) copolymer, poly (fatty acid-caprolactone) copolymer, and the blend of one or more of their derivatives.

According to further features in preferred embodiments of the invention, the biodegradable material is made from nature polymer, including collagen, chitin, chitosan, gelatin or alginate, and the blend of one or more of their derivatives.

The radiopaque material may incorporate effective components of vascular contrast agents. These components may incorporate one or more of the following materials: residues of freeze-drying of an ionic contrast agent, such as diatrizoate; and nonionic contrast agent, such as iohexol, iopromide, iopamidol, and the nonionic dimer iotrolan.

The density of the embolic microsphere is from 0.8 g/cm³ to 1.65 g/cm³, and preferred specific density of the embolic microsphere is from 1.0 g/cm³ to 1.35g/cm³.

The diameter of these microspheres lies in the range of 10µm to 1500µm. These can be subdivided into many grades such as: 10 µm ∼ 150 µm, 100 µm ∼ 350 µm, 200 µm∼ 500 µm, 400 µm∼ 650 µm, 500 µm ∼ 750 µm, 650 µm ∼ 950 µm, 850 µm ∼ 1100 µm, 1000 µm ∼ 1350 µm and 1250 µm ∼ 1500 µm.

The above mentioned radiopaque, biodegradable, therapeutic agent incorporating, embolic microspheres have the following advantages:
- Good visibility under X-ray, beneficial for ensuring accurate delivery of the embolic microspheres to the correct site.
- Controllable biodegradation rate to prevent unwanted or excessive damage to organs.
- Can consist of non-ferrous material to allow MRI examination post embolization.
- Selection of the appropriate specific gravity for the microspheres allows them to be suspended in the blood, to be taken to the targeted peripheral vessels for embolization.
- The avoidance of back flow allows accurate embolization of the target vessels.
- The diameter of the microsphere is controllable for appropriate vessel sizing.
- The resultant products are easily prepared.

In summary, an ideal vascular intervention embolic material is described, especially suited for targeting peripheral vascular vessels for embolization and/or delivery of controlled release of antineoplastic agents or anticancer drugs.

### Detailed Description of the Invention

### Example 1: Radiopaque polylactide (PLA) vascular embolic material

Vascular contrast agent, such as diatrizoate which is an ionic contrast agent, is purchased from the market. The contrast agent is processed be vacuum freeze-drying, and the residues are used as the imaging material. PLA is used as the biodegradable material, and the radiopaque microsphere can be fabricated by commonly known processes.

There are many examples of processes to prepare drug-loading microsphere with PLA as the coating materials. YANG Fan, et al, "Study on the Ciprofloxacin Polyactic Acid Microspheres" published in Journal of China Pharmaceutical University, 2004, 35: 207-210. In the article, YANG tried to optimize the preparation technology of ciprofloxaxacin polylactic acid microspheres through orthogonal test. ZHAO Rui-Ling, et al, "Study on preparation of adriamycin polylactic acid microspheres and release behavior in vitro" published in Chinese Hospital Pharmacy Journal, 2004 Feb., Vol. 24, No. 2, p74-75. In the article, ZHAO discloses that adriamycin polylactic acid microspheres were prepared by emulsifying solvent - evaporation process. LI Yan, et al, "Study on development of some factors for preparing microspheres" published in Foreign Medical Sciences (Section on Pharmacy), 2001 Jun., Vol. 28, No. 3, p164-167. In the article, LI introduces some factors for preparing microspheres.

In this example, an improved emulsification and solvent-evaporation method is used to fabricate a developable PLA microsphere-base vascular embolic agent.

PLA is dissolved in organic solvents (e.g. dichloromethane), and a radiopaque agent is dispersed into the solvent. The above mixture is added to a PVA solution drop by drop while stirring to emulsify. Then, the primary emulsification solution is poured into a large volume of PVA solution under a low concentration to evaporate the organic solvent, centrifuged, filtered, washed, dried, and sterilized. PLA microspheres incorporating radiopaque agents are thus obtained.

Other contrast agents may be used besides the diatrizoate described in this example. These contrast agents may be selected from nonionic contrast agents, such as iohexol, iopromide, iopamidol, or the nonionic dimer iotrolan.

### Example 2: Radiopaque poly(lactic-co-glycolic acid) (PLGA) vascular embolic microspheres

The second embodiment of the radiopaque biodegradable vascular embolic microsphere is generally similar to the first embodiment with the difference that in this example, the biodegradable material is PLGA rather than PLA.

The radiopaque materials are selected from the residues by freeze-drying of one of the following materials: ionic contrast agent diatrizoate, nonionic contrast agent iohexol, iopromide and iopamidol, and non-ionic dimmer iotrolan.

The developable PLA and PLGA microsphere in present invention are prepared by emulsification and solvent-evaporation method and phase separation-aggregation method.

In addition, a new method such as supercritical fluid technology can also be adopted. The solution which incorporates biodegradable material and developable material is atomized and injected into the incorporateer incorporating compressed CO₂. Because of the extraction and diffusion of organic solvents in CO₂, the polymers precipitate and the radiopaque PLA or PLGA microspheres can be obtained. The resultant microspheres have better mobility and appearance, and the amount of surfactant and residual solvents are reduced.

### Example 3: Radiopaque poly(glycolic acid-co-caprolactone) copolymer vascular embolic microspheres

The third embodiment of the radiopaque biodegradable vascular embolic microsphere is generally similar to the first embodiment with the difference that in this example, the biodegradable material is poly(glycolic acid-co-caprolactone) copolymer.

The radiopaque materials are selected from the residues by freeze-drying of one of the following materials: ionic contrast agent diatrizoate, nonionic contrast agent iohexol, iopromide and iopamidol, and non-ionic dimmer iotrolan.

The microspheres are prepared by emulsification and solvent-evaporation method and phase separation-aggregation method, or CO₂ supercritical fluid technology.

### Example 4: Radiopaque chitosan vascular embolic microspheres

The fourth embodiment of the radiopaque biodegradable vascular embolic microsphere is generally similar to the first embodiment with the difference that in this example, the biodegrade material is chitosan.

The radiopaque materials are the residues by freeze-drying of ionic contrast agent diatrizoate.

The microspheres are prepared by emulsification and solvent-evaporation method, phase separation-aggregation method or CO₂ supercritical fluid technology.

### Example 5: Radiopaque gluten vascular embolic microspheres

The fifth embodiment of the radiopaque biodegradable vascular embolic microsphere is generally similar to the first embodiment with the difference that in this example, the biodegrade material is gluten.

The radiopaque materials are the residues by freeze-drying of ionic contrast agent diatrizoate. The microspheres are prepared by emulsification and solvent-evaporation method and phase separation-aggregation method, or CO₂ supercritical fluid technology.

In above examples 1-5, the biodegradable material may incorporate the radiopaque material to form granule structure in the following three types: first, the radiopaque material is dispersively distributed in the embolic microsphere which is made of the biodegradable material; second, the radiopaque material is encapsulated by the biodegradable material to form the granular structure; third, the radiopaque material is adsorbed in the embolic microsphere which is made of the biodegradable material.

### Example 6: Radiopaque, vascular, embolic PLA microspheres incorporating therapeutic agent(s)

Vascular contrast agent is purchased from the market, such as diatrizoate which is a kind of ionic contrast agent, processed by vacuum freeze-drying, and the residues are used as the imaging materials.

Therapeutic agents such as one of the following, or combination of two or more of the antineoplastic drugs are chosen: carboplatin, cisplatin, paclitaxel, docetaxel, oxaliplatin, cyclophosphamide, ifosfamide, doxorubicin, pegylated liposomal doxorubicin, epirubicin, topotecan, irinotecan, etoposide, bleomycin, fluorouracil, gemcitabine, vincristine, actinomycin, and paclitaxel, and their derivatives, as well as any combination thereof.

PLA is used as the biodegradable material. These PLA microspheres which incorporate radiopaque materials and antineoplastic agents are prepared by commonly known processes.

In this example, a improved emulsification-solvent evaporation method is used to prepare the radiopaque, vascular, embolic PLA microspheres. Additional details are as follows.

PLA is dissolved in organic solvents (e.g. dichloromethane), and a radiopaque agent is dispersed into the solvent. The mixture is added to a PVA solution drop by drop while stirring to emulsify. Then, the primary emulsification solution is poured into a large volume of PVA solution under a low concentration to evaporate the organic solvent, centrifuged, filtered, washed, dried, and sterilized. PLA microspheres incorporating radiopaque agents are thus obtained.

In this example, the radiopaque materials also could be selected from the residues, by freeze-drying, of ionic contrast agent diatrizoate, nonionic contrast agent iohexol, iopromide and iopamidol, and non-ionic dimer iotrolan.

### Example 7: Radiopaque, vascular, embolic PLGA microspheres incorporating therapeutic agent(s)

The seventh embodiment of the radiopaque biodegradable vascular embolic microsphere is generally similar to the sixth embodiment with the difference that in this example, the biodegradable material is PLGA.

The radiopaque material is selected from one of the residues, by freeze-drying, of ionic contrast agent diatrizoate, nonionic contrast agent iohexol, iopromide and iopamidol, and non-ionic dimer iotrolan.

Therapeutic agents such as one of the following, or combination of two or more of the antineoplastic drugs are chosen: carboplatin, cisplatin, paclitaxel, docetaxel, oxaliplatin, cyclophosphamide, ifosfamide, doxorubicin, pegylated liposomal doxorubicin, epirubicin, topotecan, irinotecan, etoposide, bleomycin, fluorouracil, gemcitabine, vincristine, actinomycin, and paclitaxel, or their derivatives.

The radiopaque PLGA microspheres in present invention are prepared by emulsification and solvent-evaporation method and phase separation-aggregation method. In addition, a new method such as supercritical fluid technology can also be used. The solution which incorporates biodegradable and radiopaque materials is atomized and injected into the incorporateer incorporating compressed CO₂ By the extraction and diffusion of organic solvents in CO₂, the polymers precipitate, and the radiopaque PLGA microspheres are obtained. The resultant microspheres have better mobility and appearance, and the amount of surfactant and residual solvents are reduced.

### Example 8: Radiopaque, vascular, embolic poly(caprolactone-glycolide) microspheres incorporating therapeutic agent(s)

The eighth embodiment of the radiopaque biodegradable vascular embolic microsphere is generally similar to the sixth embodiment with the difference that in this example, the biodegradable material is poly(caprolactone-caprolactone-glycolide).

The radiopaque material is selected from one of the residues, by freeze-drying, of ionic contrast agent diatrizoate, nonionic contrast agent iohexol, iopromide and iopamidol, and non-ionic dimer iotrolan.

Therapeutic agents such as one of the following, or combination of two or more of the antineoplastic drugs are chosen: carboplatin, cisplatin, paclitaxel, docetaxel, oxaliplatin, cyclophosphamide, ifosfamide, doxorubicin, pegylated liposomal doxorubicin, epirubicin, topotecan, irinotecan, etoposide, bleomycin, fluorouracil, gemcitabine, vincristine, actinomycin, and paclitaxel, or their derivatives.

The microspheres are prepared by emulsification and solvent-evaporation method and phase separation-aggregation method, or CO₂ supercritical fluid technology.

### Example 9: Radiopaque, vascular, embolic chitosan microspheres incorporating cisplatin

The ninth embodiment of the radiopaque biodegradable vascular embolic microsphere is generally similar to the sixth embodiment with the difference that in this example, the biodegradable material is chitosan.

The radiopaque material is the residues by freeze-drying of ionic contrast agent diatrizoate.

The microspheres are prepared by emulsification and solvent-evaporation method and phase separation-aggregation method or CO₂ supercritical fluid technology.

### Example 10: Radiopaque, vascular, embolic chitosan microspheres incorporating carboplatin

The tenth embodiment of the radiopaque biodegradable vascular embolic microsphere is generally similar to the sixth embodiment with the difference that in this example, the biodegrade material is carboplatin. The radiopaque material is the residues by freeze-drying of ionic contrast agent diatrizoate.

The antineoplastic agent is carboplatin. The microspheres are prepared by emulsification and solvent-evaporation method and phase separation-aggregation method, or CO₂ supercritical fluid technology.

For flexibility and optimizing patient treatment, the degradation rate of these radiopaque microspheres related in the present invention can be controlled.

In above examples 6-10, the biodegradable material incorporates the radiopaque material and the therapeutic agent to form granule structure in the following three types: first, the radiopaque material and the therapeutic agent are dispersively distributed in the embolic microsphere which is made of the biodegradable material; second, the radiopaque material and the therapeutic agent are encapsulated by the biodegradable material to form the granular structure; third, the radiopaque material and the therapeutic agent are adsorbed in the embolic microsphere which is made of the biodegradable material.

Additionally, by changing the molecular weights of polymers or their chemical compositions of the microspheres, degradation rates such as 7 days, 15 days, 30 days, 60 days, and 180 days are achievable.

It should be noted that the structure, described and made public in this text, can be replaced with other structure(s) owning the same function. Meanwhile, the examples introduced by the invention are not the only structure that does carry out the invention. Although this invention has given introduction and elucidation of the implementation example, the technician in this field are clearly know that this is just an illustration with example. The technician in this field can make numerous changes, improvements and replacements. However, it can't be divorced from this invention. Therefore, we should limit protection ranges of the invention according to the spirit and scope of claims enclosed invention.

### Technology Field

The present invention relates to a vascular embolic material, particularly relates to a radiopaque biodegradable vascular embolic microsphere, and relates to an embolic vascular microsphere which is biodegradable, and incorporates a therapeutic agent or agents or a drug or drugs.

### Background Art

Various materials have been proposed for interventional vascular embolization. Commonly used examples include: polyvinyl alcohol (PVA), sodium alginate, gelatin sponge, surgical sutures, stainless steel or platinum coils, detachable balloons, cyanoacrylates, anhydrous ethanol, iodized oil, sodium morrhuate, et al. Positive clinical results have been obtained using stainless steel coils, surgical sutures, and gelatin sponges as embolic agents in vasculars. However, the long term curative effects have been limited due to the establishment of collateral circulation. The embolic agents which are liquid, such as cyanoacrylates, anhydrous ethanol, iodized oil, and sodium morrhuate, may cause non-target embolization and are difficult to control during the procedure. In addition, the embolic agents that are non-radiopaque; such as polyvinyl alcohol, sodium alginate microspheres, gelatin sponges, and surgical sutures, are difficult to assess the proper delivery of these materials to the site.

### Contents of the Invention

The present invention describes a radiopaque, biodegradable, embolic microspheres for use in the vascular system. These microspheres, readily visible under X-ray, can be delivered or drift with blood flow to a peripheral vessel or site, and can be manufactured using a simple process.

According to the present invention, there is provided a radiopaque biodegradable vascular embolic microsphere comprising of a biodegradable material and a radiopaque material which can be both delivered to the vascular system. The biodegradable material incorporates the radiopaque material to form granule structure.

The present invention also describes a radiopaque, biodegradable, embolic microsphere which incorporates a therapeutic agent. This microsphere can be delivered to the targeted site under X-ray guidance. The microsphere can then embolize the site, and release the therapeutic agent directly at the targeted site while degrading *in vivo*.

According to the present invention, there is also provided a radiopaque biodegradable vascular embolic microsphere incorporating therapeutic agent(s) comprising of a biodegradable material, a radiopaque material, and therapeutic agent(s), which can be all delivered to the vascular system. The biodegradable material incorporates the radiopaque material and the therapeutic agent to form granule structure.

The preferred therapeutic agent is an antineoplastic agent. These agents can include the following drugs: carboplatin, cisplatin, docetaxel, oxaliplatin, cyclophosphamide, ifosfamide, doxorubicin, pegylated liposomal doxorubicin, epirubicin, topotecan, irinotecan, etoposide, bleomycin, fluorouracil, gemcitabine, vincristine, actinomycin, and paclitaxel, or one of their derivatives, as well as any combination thereof.

## Claims

1. A radiopaque biodegradable vascular embolic microsphere comprising of a biodegradable material and a radiopaque material which can be both delivered to the vascular system, wherein said biodegradable material incorporates said radiopaque material to form granule structure.

2. The radiopaque biodegradable vascular embolic microsphere according to claim 1, wherein said biodegradable material is made from polyesters, including polylactide, poly (lactide-co-glycolide), poly (lactide-ether) copolymer, and the blend of one or more of their derivatives.

3. The radiopaque biodegradable vascular embolic microsphere according to claim 1, wherein said biodegradable material is made from polycaprolactone, including polycaprolactone, poly (caprolactone-ether) block copolymer, polycaprolactone copolymer, poly (caprolactone-ether-lactide) copolymer, poly (glycolide-L-lactide) copolymer, poly (glycolide-L-lactide-caprolactone) copolymer, poly (caprolactone-caprolactone-glycolide) copolymer, poly (fatty acid-caprolactone) copolymer, and the blend of one or more of their derivatives.

4. The radiopaque biodegradable vascular embolic microsphere according to claim 1, wherein said biodegradable material is made from nature polymer, including collagen, chitin, chitosan, gelatin or alginate, and the blend of one or more of their derivatives.

5. The radiopaque biodegradable vascular embolic microsphere according to claim 1, wherein said radiopaque material incorporates effective components of vascular contrast agents.

6. The radiopaque biodegradable vascular embolic microsphere according to claim 5, wherein said radiopaque material incorporates at least one of the following materials: vacuum freeze-dried ionic contrast agent, such as diatrizoate; and vacuum freeze-dried nonionic contrast agent, such as iohexol, iopromide, iopamidol, or dimmer iotrolan.

7. The radiopaque biodegradable vascular embolic microsphere according to claim 1, wherein the density of said embolic microsphere is from 0.8 g/cm³ to 1.65 g/cm³, and preferred specific density of said embolic microsphere is from 1.0 g/cm³ to 1.35g/cm³.

8. The radiopaque biodegradable vascular embolic microsphere according to claim 1, wherein the diameter of said embolic microsphere is from 10 µm to 1500 µm, and can be subdivided into the following grades: 10 µm ∼150 µm, 100 µm ∼350 µm, 200 µm ∼500 µm, 400 µm ∼650 µm, 500 µm ∼750 µm, 650 µm ∼950 µm, 850 µm ∼1100 µm, 1000 µm ∼1350 µm, and 1250 µm ∼ 1500 µm.

9. A radiopaque biodegradable vascular embolic microsphere incorporating therapeutic agent(s) comprising of a biodegradable material, a radiopaque material, and therapeutic agent(s), which can be all delivered to the vascular system, wherein said biodegradable material incorporates said radiopaque material and said therapeutic agent to form granule structure.

10. The radiopaque biodegradable vascular embolic microsphere incorporating therapeutic agent(s) according to claim 9, wherein said therapeutic agent is an anti-neoplastic agent.

11. The radiopaque biodegradable vascular embolic microsphere incorporating therapeutic agent(s) according to claim 10, wherein said therapeutic agent is selected from one of the following anti-neoplastic agent: carboplatin, cisplatin, paclitaxel, docetaxel, oxaliplatin, cyclophosphamide, ifosfamide, doxorubicin, pegylated liposomal doxorubicin, epirubicin, topotecan, irinotecan, etoposide, bleomycin, fluorouracil, gemcitabine, vincristine, actinomycin, and paclitaxol, and their derivatives, as well as any combination thereof.

12. The radiopaque biodegradable vascular embolic microsphere incorporating therapeutic agent(s) according to claim 9, wherein said biodegradable material is selected from polylactide, including polylactde, poly (lactide-co-glycolide), poly (lactde-ether) copolymer, and the blend of one or more of their derivatives.

13. The radiopaque biodegradable vascular embolic microsphere incorporating therapeutic agent(s) according to claim 9, wherein said biodegradable material is selected from polycaprolactone, including polycaprolactone, poly (caprolactone-ether) block copolymer, polycaprolactone copolymer, poly (caprolactone -ether-lactide) copolymer, poly (glycolide-L-lactide) copolymer, poly (glycolide-L-lactide-caprolactone) copolymer, poly (caprolactone-glycolide) copolymer, poly (fatty acid-caprolactone) copolymer, and the blend of one or more of their derivatives.

14. The radiopaque biodegradable vascular embolic microsphere incorporating therapeutic agent(s) according to claim 9, wherein said biodegradable material is selected from nature polymer, including collagen, chitin, chitosan, gelatin, alginate, and the blend of one or more of their derivatives.

15. The radiopaque biodegradable vascular embolic microsphere incorporating therapeutic agent(s) according to claim 9, wherein said radiopaque material incorporates effective components of vascular contrast agents.

16. The radiopaque biodegradable vascular embolic microsphere incorporating therapeutic agent(s) according to claim 9, wherein said radiopaque material incorporates at least one of the following materials: vacuum freeze-dried ionic contrast agent, such as diatrizoate; and vacuum freeze-dried nonionic contrast agent, such as iohexol, iopromide, iopamidol, or dimmer iotrolan.

17. The radiopaque biodegradable vascular embolic microsphere incorporating therapeutic agent(s) according to claim 1, wherein the density of said embolic microsphere is from 0.8 g/cm³ to 1.65 g/cm³, and preferred specific density of said embolic microsphere is from 1.0 g/cm³ to 1.35g/cm³.

18. The radiopaque biodegradable vascular embolic microsphere incorporating therapeutic agent(s) according to claim 1, wherein the diameter of said embolic microsphere is from 10 µm to 1500 µm, and can be subdivided into the following grades: 10 µm ∼150 µm, 100 µm ∼350 µm, 200 µm ∼500 µm, 400 µm ∼650 µm, 500 µm ∼750 µm, 650 µm ∼950 µm, 850 µm ∼1100 µm, 1000 µm ∼1350 µm, and 1250 µm ∼1500 µm.
